(19) Europäisches Patentamt
European Patent Office
Office européen des brevets

(11) Veröffentlichungsnummer : **0 011 208**
**B1**

(12) # EUROPÄISCHE PATENTSCHRIFT

(45) Veröffentlichungstag der Patentschrift : **29.07.81**

(51) Int. Cl.³ : **C 07 C 47/575**, C 07 C 45/29

(21) Anmeldenummer : **79104308.6**

(22) Anmeldetag : **05.11.79**

(54) **Verfahren zur Herstellung von 3-Phenoxy-benzaldehyden.**

(30) Priorität : **18.11.78 DE 2850180**

(43) Veröffentlichungstag der Anmeldung : **28.05.80 (Patentblatt 80/11)**

(45) Bekanntmachung des Hinweises auf die Patenterteilung : **29.07.81 Patentblatt 81/30**

(84) Benannte Vertragsstaaten : **BE CH DE FR GB IT NL**

(56) Entgegenhaltungen :
**DE - A1 - 2 620 254**
**DE - A1 - 2 704 512**
**TETRAHEDRON LETTERS, Band 1978, Nr. 18 Oxford, New York, Paris, Frankfurt D. PLETCHER et al. : "A procedure for the oxidation of alcohols to aldehydes based on phase transfer catalysis", Seiten 1 601 bis 1 602**

(73) Patentinhaber : **BAYER AG**
**Zentralbereich Patente, Marken und Lizenzen**
**D-5090 Leverkusen 1, Bayerwerk (DE)**

(72) Erfinder : **Böhm, Siegfried, Dr.**
**Silesiusstrasse 80**
**D-5000 Köln 80 (DE)**
Erfinder : **Klein, Alfons, Ing.-Grad.**
**Virchowstrasse 9**
**D-4000 Düsseldorf (DE)**
Erfinder : **Wedemeyer, Karlfried, Dr.**
**Bilharzstrasse 7**
**D-5000 Köln 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 011 208 B1

Verfahren zur Herstellung von 3-Phenoxy-benzaldehyden

Die Erfindung betrifft ein Verfahren zur Herstellung von 3-Phenoxy-benzaldehyden aus 3-Phenoxy-benzylalkoholen bzw. 3-Phenoxy-benzylchloriden.

Aus der DE-OS 2 741 764 und der DE-OS 2 704 512 ist bekannt, ein Gemisch von 3-Phenoxy-benzylchlorid und 3-Phenoxy-benzalchlorid in einer ersten Reaktionsstufe mit Ammoniak und Formaldehyd oder Hexamethylentetramin umzusetzen und dann in einer zweiten Reaktionsstufe unter sauren Bedingungen zum 3-Phenoxy-benzaldehyd zu hydrolysieren. In der zweiten Reaktionsstufe wird das Reaktionsprodukt der ersten Stufe in einer Wasser, Essigsäure und konzentrierte Salzäure enthaltenden Lösung unter Rückfluß gekocht. Hierbei treten bei der technischen Durchführung Korrosionsprobleme auf.

Es wurde ein Verfahren zur Herstellung von 3-Phenoxy-benzaldehyden aus 3-Phenoxy-benzylalkoholen gefunden, das dadurch gekennzeichnet ist, daß man 3-Phenoxy-benzyl-alkohole der Formel

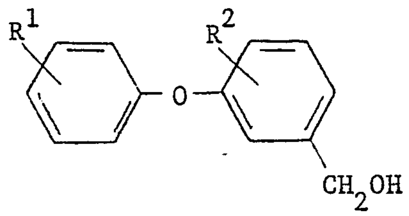

(I)

worin $R^1$ und $R^2$ gleich oder verschieden sind und für Wasserstoff oder Halogen stehen, in Gegenwart von wässriger Schwefelsäure im Temperaturbereich von 50 bis 125 °C mit einer wässrigen Lösung eines Dichromats oxydiert.

Das erfindungsgemäße Verfahren kann am Beispiel der folgenden Reaktionsgleichung erläutert werden :

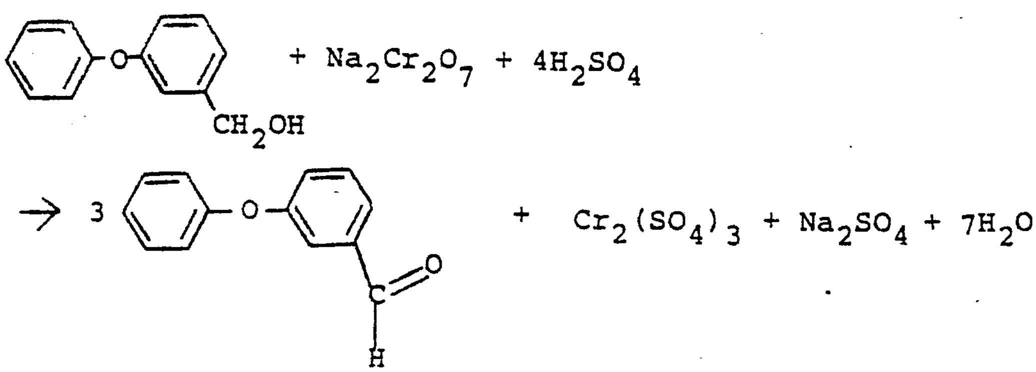


Halogen der Formel (I) kann Fluor, Chlor, Brom oder Jod, bevorzugt Fluor oder Chlor sein.

Die 3-Phenoxy-benzylalkohole für das erfindungsgemäße Verfahren sind bekannt, und können vorteilhafterweise durch Hydrolyse von 3-Phenoxy-benzylchloriden im Temperaturbereich von 140 bis 210 °C unter Druck hergestellt werden.

Das erfindungsgemäße Verfahren wird im Temperaturbereich von 50 bis 125 °C, bevorzugt 80 bis 100 °C, durchgeführt. Das erfindungsgemäße Verfahren wird in allgemeinen unter Normaldruck durchgeführt.

Als Dichromat für das erfindungsgemäße Verfahren wird im allgemeinen ein Alkalidichromat verwandt. Beispielsweise sei das Natrium- und das Kaliumdichromat genannt. Selbstverständlich ist es auch möglich, Gemische verschiedener Dichromate in das erfindungsgemäße Verfahren einzusetzen.

Im allgemeinen werden für das erfindungsgemäße Verfahren 90 bis 130 Mol, bevorzugt 105 bis 120 Mol, des Dichromats bezogen auf 1 Mol des Benzylalkohols eingesetzt.

Für das erfindungsgemäße Verfahren wird das Dichromat im allgemeinen in einer 10 Gew-% bis gesättigten Lösung, bevorzugt 20 bis 60 Gew.-% wässriger Lösung eingesetzt.

Der Gehalt an Schwefelsäure ($H_2SO_4$) für das erfindungsgemäße Verfahren kann in weiten Grenzen schwanken. Er beträgt im allgemeinen 2,5 bis 10 Mol, bevorzugt 3 bis 7 Mol, bezogen auf 1 Mol des Dichromats.

Für das erfindungsgemäße Verfahren wird im allgemeinen 10 bis 70 gew.-%ige bevorzugt 20 bis 60 gew.-%ige, wässrige Schwefelsäure eingesetzt.

Das erfindungsgemäße Verfahren wird vorteilhafterweise in Gegenwart eines inerten, nicht wasserlöslichen Lösungsmittels durchgeführt. Solche Lösungsmittel können beispielsweise aliphatische oder aromatische Kohlenwasserstoffe, wie z.B. Hexan, iso-Octan, Benzol, Xylol oder Xylole sein.

In einer bevorzugten Ausführungsform des erfindungsgemäßen Verfahrens wird in einer ersten

Reaktionsstufe ein Gemisch aus seitenkettenchlorierten 3-Phenoxy-toluolen der Formel

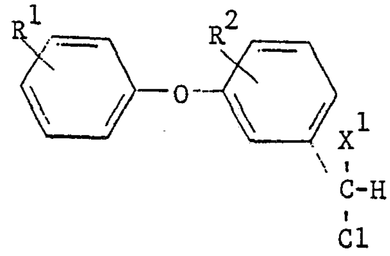

(II)

worin $X^1$ für Wasserstoff oder Chlor steht, und $R^1$ und $R^2$ gleich oder verschieden sind und die obengenannte Bedeutung haben, im Temperaturbereich von 140 bis 210 °C unter Druck zu einer 3-Phenoxy-benzylalkohole der Formel (I) enthaltenden Mischung hydrolysiert, die dann in einer zweiten Reaktionsstufe in Gegenwart von wäßriger Schwefelsäure im Temperaturbereich von 50 bis 125 °C mit einer wäßrigen Lösung eines Dichromats oxydiert wird.

Seitenkettenchlorierte 3-Phenoxy-toluole sind an sich bekannt (DE-OS 2 704 512). Sie können beispielsweise durch Chlorierung der entsprechenden 3-Phenoxy-toluole in Tetrachlorkohlenstoff in Gegenwart eines Radikalinitiators oder unter Belichtung hergestellt werden. Bei einer solchen Chlorierung erhält man neben dem 3-Phenoxy-benzylchlorid, auch 3-Phenoxy-benzalchlorid, nicht umgesetztes 3-Phenoxy-toluol und gegebenenfalls 3-Phenoxy-benzotrichlorid.

Für das erfindungsgemäß bevorzugte Verfahren ist es möglich, diese Chlorierungsgemische direkt ohne eine Vortrennung einzusetzen.

Vorzugsweise setzt man in das erfindungsgemäß bevorzugte Verfahren seitenkettenchlorierte 3-Phenoxy-toluole ein, die dadurch entstehen, daß man die Seitenchlorierung abbricht, wenn ein Gemisch entstanden ist, das bis zu 20 Gew.-% 3-Phenoxy-benzalchlorid enthält.

Die Hydrolyse nach der bevorzugten Ausführungsform des erfindungsgemäßen Verfahrens eingesetzten seitenkettenchlorierten 3-Phenoxy-toluolen in der 3-Phenoxy-benzyl-alkohole enthaltenden Mischung kann in Gegenwart von wäßrigen Basen durchgeführt werden.

Als wäßrige Basen für das erfindungsgemäß bevorzugten Verfahren werden im allgemeinen wäßrige Lösungen und/oder Suspensionen von Alkali- oder Erdalkali-Oxyden, Hydroxyden oder Carbonaten verwandt. Beispielsweise seien die folgenden Basen genannt: Natriumhydroxyd, Natriumcarbonat, Natriumhydrogencarbonat, Kaliumhydroxyd, Kaliumcarbonat, Kaliumhydrogencarbonat, Magnesiumoxyd, Magnesiumhydroxyd, Magnesiumcarbonat, Calciumoxyd, Calciumhydroxyd und Calciumcarbonat. Bevorzugte Basen für das erfindungsgemäße Verfahren sind Natriumhydroxyd, Natriumcarbonat, Calciumoxyd und Magnesiumoxid.

Die Alkali- bzw. Erdalkali-Verbindungen können einzeln oder im Gemisch verwendet werden. Es ist auch möglich, die Umsetzung mit einer dieser Verbindungen zu beginnen und im Verlauf der Reaktion eine andere zuzusetzen.

Für das bevorzugte Verfahren kann die Menge der Base in weiten Grenzen schwanken. Im allgemeinen kann man 50 bis 150 Mol-%, vorzugsweise 75 bis 120 Mol-%, der dem Gehalt an Seitenkettenchlor im 3-Phenoxy-benzylchlorid äquivalenten Menge der Base einsetzen.

Zur Hydrolyse nach dem erfindungsgemäß bevorzugten Verfahren verwendet man im allgemeinen die 0,5 bis 10-fache, vorzugsweise die 1,5 bis 4-fache Menge Wasser, bezogen auf das eingesetzte Chlorierungsprodukt:

In einer besonderen Ausführungsform des erfindungsgemäß bevorzugten Verfahrens ist es möglich, die Base nicht zu Beginn der Hydrolyse, sondern erst zu einem späteren Zeitpunkt zuzusetzen. Beispielsweise gibt man nach 50 bis 85 Mol-% Umsatz, bezogen auf den Gehalt an Seitenkettenchlor im 3-Phenoxy-benzylchlorid, die Base hinzu.

Es ist aber auch möglich, die Hydrolyse ohne Zusatz einer Base durchzuführen.

Es ist aber auch möglich, die Hydrolyse in mehreren Stufen durchzuführen. So kann man beispielsweise nach etwa 40 bis 70 % des Gesamtumsatzes abbrechen, die wäßrige Phase entfernen und mit einer neuen wäßrigen Phase zu Ende führen.

Die Hydrolyse nach dem erfindungsgemäß bevorzugten Verfahren wird in dem Temperaturbereich von 140 bis 210 °C, vorzugsweise von 150 bis 190 °C, durchgeführt. Die Umsetzung nach diesem Verfahren ist im allgemeinen nach 2 bis 7 Stunden beendet.

Die Hydrolyse nach dem erfindungsgemäß bevorzugten Verfahren wird bei Überdruck, im allgemeinen im Druckbereich von 2 bis 100 bar, bevorzugt von 3 bis 50 bar, durchgeführt. Das erfindungsgemäß bevorzugte Verfahren wird zweckmäßigerweise in einem Druckgefäß, bevorzugt einem Autoklaven, durchgeführt. Das erfindungsgemäß bevorzugte Verfahren wird vorzugsweise bei dem Druck der Reaktionsmischung durchgeführt, der sich in dem Druckgefäß bei der gewählten Reaktionstemperatur einstellt.

Das erfindungsgemäße Verfahren kann beispielsweise wie folgt durchgeführt werden:

Gegebenenfalls werden in einer ersten Reaktionsstufe die seitenkettenchlorierten 3-Phenoxy-toluole,

Wasser und gegebenenfalls eine Base in einem Autoklaven gegeben und auf die Reaktionstemperatur erhitzt. Nach Beendigung der Reaktion kann das Reaktionsprodukt nach an sich bekannten Methoden aufgearbeitet werden. Beispielsweise trennt man die wäßrige Schicht von der organischen Phase ab und isoliert aus der organischen Phase ein 3-Phenoxy-benzylalkohol enthaltendes Produkt.

Dieses Produkt wird in einer zweiten Reaktionsstufe erfindungsgemäß zu dem 3-Phenoxy-benzaldehyd oxidiert.

Zur Durchführung der Oxydation kann man den 3-Phenoxybenzylalkohol durch Rühren mit einer wäßrigen Chromatlösung emulgieren und dann anschließend die Schwefelsäure zutropfen.

Man kann jedoch auch den 3-Phenoxy-benzylalkohol mit der verdünnten Schwefelsäure mischen und dann mit der wäßrigen Chromatlösung versetzen.

Nach Beendigung der Reaktion kann man den entstandenen 3-Phenoxy-benzaldehyd beispielsweise durch Extraktion, vorzugsweise mit dem gegebenenfalls zu der Umsetzung zugesetzten inerten, nicht wäßrigen Lösungsmittel, extrahieren. Nach Trocknen des Extraktionsgemisches erhält man den 3-Phenoxy-benzaldehyd durch Destillation. Es ist selbstverständlich auch möglich, den Aldehyd über die entsprechende Bisulfitverbindung zu isolieren.

Es ist überraschend, daß sich nach dem erfindungsgemäßen Verfahren mit Hilfe der an sich bekannten Oxydation mit Chromat aus 3-Phenoxy-benzylalkohol die 3-Phenoxy-benzaldehyde herstellen lassen, da aus Houben-Weyl, Band 7/1, Seite 171, (1954) bekannt ist, daß primäre Alkohole, die in ihrem Molekül noch Äthergruppen enthalten, durch Chromat zerstört werden.

Nach dem erfindungsgemäßen Verfahren können 3-Phenoxy-benzaldehyde der Formel

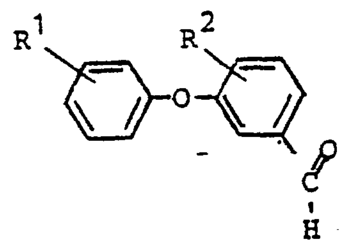

(III)

worin $R^1$ und $R^2$ gleich oder verschieden sind und die oben angegebene Bedeutung haben, hergestellt werden.

Die Herstellung der 3-Phenoxy-benzaldehyde nach dem erfindungsgemäßen Verfahren erfolgt mit großen Ausbeuten und ohne Ausfall von schwer aufarbeitbaren Nebenprodukten. Vorteilhafterweise treten bei der Umsetzung keine Korrosionsprobleme auf.

Der 3-Phenoxy-benzaldehyd ist ein Zwischenprodukt für Pflanzenschutzmittel (Nachrichten aus Chemie, Technik und Laboratorium *26*, 120 (1978)).

## Beispiel 1

Ein Chlorierungsprodukt folgender Zusammensetzung wird hydrolysiert :

| | |
|---|---|
| Unbekannte Anteile | 1,1 % |
| 3-Phenoxy-toluol | 46,7 % |
| kernchlorierte Anteile | 0,7 % |
| 3-Phenoxy-benzylchlorid | 46,9 % |
| Unbekannte Anteile | 1,0 % |
| 3-Phenoxy-benzalchlorid | 3,6 % |

Der Gehalt an Seitenkettenchlor beträgt 10,1 %.

150 g dieses Chlorierungsgemisches

37,5 g 50 %ige Natronlauge und

285 g Wasser werden in einem Autoklaven 3 Stunden bei 180 °C gerührt. Nach dem Abkühlen trennt man von der wäßrigen Schicht und destilliert die organische Phase über eine Destillationsbrücke. Bei $Kp_{1,0}$ bis zu einer Temperatur von 190 °C erhält man 124,6 g Destillat. Es verbleibt ein Rückstand von 10,3 g.

Das Destillat ist wie folgt zusammengesetzt :

| | |
|---|---|
| Unbekannte Anteile | 0,4 % |
| 3-Phenoxy-toluol | 48,9 % |
| kernchlorierte Anteile | 0,6 % |
| 3-Phenoxy-benzaldehyd | 4,6 % |
| Unbekannte Anteile | 0,4 % |

| | |
|---|---|
| 3-Phenoxy-benzylchlorid | 0,2 % |
| 3-Phenoxy-benzylalkohol | 44,9 % |

Beispiel 2

150 g eines Chlorierungsproduktes der Zusammensetzung wie in Beispiel 1
13,3 g Calciumoxid und
300 g Wasser werden 3 Stunden im Autoklaven bei 180 °C gerührt. Nach der Aufarbeitung, die analog derjenigen des Beispiels 1 erfolgt, erhält man ein Destilliat von 124,2 g.
Es bleibt ein Rückstand von 6,5 g.
Zusammensetzung des Destillates :

| | |
|---|---|
| Unbekannte Anteile | 0,1 % |
| 3-Phenoxy-toluol | 51,5 % |
| kernchlorierte Anteile | 0,5 % |
| 3-Phenoxy-benzaldehyd | 3,1 % |
| Unbekannte Anteile | 0,1 % |
| 3-Phenoxy-benzylchlorid | 0,1 % |
| 3-Phenoxy-benzylalkohol | 44,6 % |

Beispiel 3

150 g eines Chlorierungsgemisches, welches folgendermaßen zusammengesetzt ist :

| | |
|---|---|
| Unbekannte Anteile | 2,3 % |
| 3-Phenoxy-toluol | 29,8 % |
| kernchlorierte Anteile | 1,3 % |
| 3-Phenoxy-benzylchlorid | 55,1 % |
| Unbekannte Anteile | 3,0 % |
| 3-Phenoxy-benzalchlorid | 6,5 % |

Der Gehalt an Seitenkettenchlor beträgt 12,0 %.
10,7 g Magnesiumoxid und
300 g Wasser werden wie in Beispiel 1 beschrieben umgesetzt und aufgearbeitet.
Das erhaltene Destillat wiegt 130,5 g
Der Rückstand wiegt 5,5 g.
Zusammensetzung des Destillates :

| | |
|---|---|
| Unbekannte Anteile | 1,2 % |
| 3-Phenoxy-toluol | 44,1 % |
| kernchlorierte Anteile | 1,2 % |
| 3-Phenoxy-benzaldehyd | 3,0 % |
| Unbekannte Anteile | 0,6 % |
| 3-Phenoxy-benzylchlorid | 0,1 % |
| 3-Phenoxy-benzylalkohol | 49,7 % |

Beispiel 4

100 g eines Chlorierungsgemisches mit folgender Zusammensetzung.

| | |
|---|---|
| Unbekannte Anteile | 1,6 % |
| p-Phenoxy-toluol | 19,1 % |
| kernchlorierte Anteile | 1,2 % |
| 3-Phenoxy-benzylchlorid | 64,1 % |
| Unbekannte Anteile | 1,2 % |
| 3-Phenoxy-benzalchlorid | 12,9 % |

Der Gehalt an Seitenkettenchlor beträgt 15,9 %

27,3 g Natriumcarbonat und
250 g Wasser werden im Autoklaven 3 Stunden bei 180 °C gerührt. Nach der Aufarbeitung, wie in Beispiel 1 beschrieben, erhält man 77,1 g Destillat und 6,7 g Rückstand.

Zusammensetzung des Destillats :

| | |
|---|---|
| Unbekannte Anteile | 0,9 % |

| | |
|---|---|
| 3-Phenoxy-toluol | 29,7 % |
| kernchlorierte Anteile | 1,2 % |
| 3-Phenoxy-benzaldehyd | 11,3 % |
| Unbekannte Anteile | 0,6 % |
| 3-Phenoxy-benzylchlorid | 0,3 % |
| 3-Phenoxy-benzylalkohol | 55,9 % |

Beispiel 5

170 g eines Chlorierungsgemisches mit folgender Zusammensetzung :

| | |
|---|---|
| Unbekannte Anteile | 0,7 % |
| 3-Phenoxy-4-fluor-toluol | 28,9 % |
| kernchlorierte Anteile | 0,3 % |
| 3-Phenoxy-4-fluor-benzylchlorid | 63,3 % |
| Unbekannte Anteile | 0,5 % |
| 3-Phenoxy-4-fluor-benzalchlorid | 6,3 % |

Der Gehalt an Seitenkettenchlor beträg 12,4 %.

53 g 50 %ige Natronlauge und

310 g Wasser werden im Autoklaven 3 Stunden bei 180 °C gerührt. Nach dem Abkühlen wird die organische Phase in Toluol aufgenommen und von der wäßrigen Schicht abgetrennt. Nach dem Abdestillieren des Toluols verbleiben:

149 g Reaktionsprodukt mit folgender Zusammensetzung :

| | |
|---|---|
| Unbekannte Anteile | 0,2 % |
| 3-Phenoxy-4-fluor-toluol | 39,3 % |
| kernchlorierte Anteile | 0,6 % |
| 3-Phenoxy-4-fluor-benzaldehyd | 2,3 % |
| Unbekannte Anteile | 0,3 % |
| 3-Phenoxy-4-fluor-benzylalkohol | 57,3 % |

Beispiel 6

In einer Rührapparatur werden 230 g $H_2O$, 135,2 g konz. Schwefelsäure und 460 g Toluol vorgelegt. Unter kräftigem Rühren gibt man 460 g eines Gemisches aus 3-Phenoxy-benzylalkohol und 3-Phenoxy-toluol nach Beispiel 4 mit einem gaschromatographisch ermittelten Gehalt an 3-Phenoxy-benzylalkohol von 55,9 % zu und erwärmt die Emulsion zum Sieden (90 °C). Man tropft bei dieser Temperatur in 40 Min. eine Lösung von 135 g $Na_2Cr_2O_7 \times H_2O$ in 92 g $H_2O$ zu, rührt 30 Min. nach, kühlt ab und trennt die Toluolphase von der Chromlauge ab. Das Toluol wird im Wasserstrahlvakuum abdestilliert und der Rückstand im Hochvakuum bei 0,5 bar destilliert. Man erhält 430,5 g eines Gemisches aus 3-Phenoxy-benzaldehyd und 3-Phenoxytoluol mit einem gaschromatographisch ermittelten Gehalt an 3-Phenoxy-benzaldehyd von 54,6 %.

Beispiel 7

In einer Rührapparatur werden 165 g $H_2O$ und 132,6 g $H_2SO_4$ vorgelegt. In der verdünnten Schwefelsäure emulgiert man durch Rühren 165 g 3-Phenoxy-benzylalkohol. Die Emulsion wird auf 90 °C erwärmt und bei dieser Temperatur innerhalb von 30 Min. eine Lösung von 87,8 g $Na_2Cr_2O_7 \times H_2O$ in 58,5 g $H_2O$ zugetropft. Man rührt 30 Min. nach und extrahiert das Reaktionsprodukt mit 165 g Toluol aus der Chromlauge. Das Toluol wird im Wasserstrahlvakuum abgedampft und der Rückstand im Hochvakuum destilliert. Man erhält 132,2 g farblosen 3-Phenoxybenzaldehyd mit einer gaschromatographisch ermittelten Reinheit von 98,2 %.

Beispiel 8

294 g eines Gemisches aus 3-Phenoxy-4-fluor-benzylalkohol und 3-Phenoxy-4-fluor-toluol nach Beispiel 5 mit einem Gehalt von 57,3 % 3-Phenoxy-4-fluor-benzylalkohol werden unter Rühren in einer Mischung aus 294 g Wasser und 128 g Schwefelsäure emulgiert. Man gibt 294 g Toluol zu und erwärmt zum Siedepunkt des Azeotrops Wasser/Toluol (90°). Bei dieser Temperatur tropft man in 30 Min. eine Lösung von 86 g $Na_2Cr_2O_7 \times H_2O$ in 57,0 $H_2O$ zu, rührt 30 Min. nach, kühlt ab und trennt die organische Phase von der Chromlauge. Man erhält 293 g organische Phase mit einem gaschromatographisch ermittelten Gehalt an 3-Phenoxy-4-fluor-benzaldehyd von 54,8 %.

## Beispiel 9

960 g eines Chlorierungsproduktes mit folgender Zusammensetzung :

| | |
|---|---|
| Unbekannte Anteile | 1,5 % |
| 3-Phenoxy-toluol | 30,3 % |
| kernchlorierte Anteile | 0,8 % |
| 3-Phenoxy-benzylchlorid | 59,1 % |
| Unbekannte Anteile | 0,8 % |
| 3-Phenoxy-benzalchlorid | 7,5 % |

307 g 50 %ige Natronlauge und

1 758 g Wasser werden im Autoklaven 3 Stunden bei 180 °C gerührt. Nach dem Abkühlen gibt man 852 g Toluol zu, säuert mit Schwefelsäure schwach an und trennt die wäßrige Schicht ab.

In einem Rührgefäß wird diese toluolische Lösung mit

852 g Wasser und

392 g konz. Schwefelsäure durch gutes Rühren emulgiert und zum Sieden erhitzt. Die Sumpftemperatur beträgt dabei konstant 90 °C. Man tropft in 30 Min. eine Lösung von

260 g Natriumdichromat in

173 g Wasser zu und läßt 30 Min. nachrühren. Nach dem Abkühlen auf 50 °C wird die wäßrige Chromlauge abgetrennt und die toluolische Lösung in einer 60-cm-Laborfüllkörperkolonne destilliert.

Nachdem das Toluol abdestilliert ist, erhält man :

| | |
|---|---|
| Frakt. 1 $Kp_{12}$ 140-157 °C | 253,6 g |
| Frakt. 2 $Kp_{12}$ 157-176 °C | 24,8 g |
| Frakt. 3 $Kp_{12}$ 176-178 °C | 378,2 g |
| Rückstand | 159,6 g |

Fraktion 1 besteht aus 3-Phenoxy-toluol, Fraktion 3 enthält 3-Phenoxy-benzaldehyd in einer Reinheit von 98,8 %.

## Beispiel 10

150 g chloriertes Material mit folgender Zusammensetzung :

| | |
|---|---|
| Unbekannte Anteile | 0,9 % |
| 3-Phenoxy-toluol | 17,9 % |
| kernchlorierte Anteile | 2,1 % |
| 3-Phenoxy-benzylchlorid | 61,3 % |
| Unbekannte Anteile | 1,3 % |
| 3-Phenoxy-benzalchlorid | 16,8 % |
| Unbekannte Anteile | 2,0 % |

Der Gehalt an Seitenkettenchlor beträgt 15,9 %.

480 g Wasser werden in einem Autoklaven 3 Stunden bei 160 °C gerührt. Man kühlt ab, trennt von der wäßrigen Schicht und rührt die organische Phase nach Zugabe von weiteren.

480 g Wasser nochmals 3 Stunden bei 160 °C.

Man erhält nach dem Abtrennen der wäßrigen Schicht 125 g einer organischen Phase mit folgender Zusammensetzung :

| | |
|---|---|
| Unbekannte Anteile | 0,8 % |
| 3-Phenoxy-toluol | 21,2 % |
| kernchlorierte Anteile | 2,6 % |
| 3-Phenoxy-benzaldehyd | 12,3 % |
| 3-Phenoxy-benzylchlorid | 0,9 % |
| Unbekannte Anteile | 1,8 % |
| 3-Phenoxy-benzylalkohol | 60,1 % |
| Unbekannte Anteile | 0,3 % |

Bezogen auf das eingesetzte 3-Phenoxy-benzylchlorid beträgt die Ausbeute an 3-Phenoxy-benzylalkohol 89 % des theoretischen Umsatzes.

## Beispiel 11

120 g chloriertes Material von der Zusammensetzung wie in Beispiel 6.

360 g Wasser werden in einem Autoklaven bei 170 °C insgesamt 7 Stunden gerührt. Nach der 1 Std. pumpt man 66 ml, nach der 2. Std. 35 ml, nach der 4. Std. 35 ml und nach der 6. Std. 30 ml einer 10 %igen Natronlauge zu. Nach dem Abkühlen trennt man die wäßrige Schicht ab und destilliert sie wie in Beispiel 2 beschrieben. Man erhält 98 g Destillat. Es verbleibt ein Rückstand von 5 g.

Das Destillat ist wie folgt zusammengesetzt :

| | |
|---|---|
| Unbekannte Anteile | 0,9 % |
| 3-Phenoxy-toluol | 19,6 % |
| kernchlorierte Anteile | 2,3 % |
| 3-Phenoxy-benzaldehyd | 14,0 % |
| 3-Phenoxy-benzylchlorid | 1,1 % |
| Unbekannte Anteile | 1,3 % |
| 3-Phenoxy-benzylalkohol | 60,3 % |
| Unbekannte Anteile | 0,4 % |

Bezogen auf eingesetztes 3-Phenoxy-benzylchlorid beträgt die Ausbeute an 3-Phenoxy-benzylalkohol 86 % des theoretischen Umsatzes.

Beispiel 12

150 g chloriertes Material von der Zusammensetzung wie in Beispiel 6.

300 g Wasser werden in einem Autoklaven bei 160 °C insgesamt 7 Stunden gerührt. Nach der 1 Stunde pumpt man 45 ml, nach der 2. Std. 24 ml und nach der 6 Std. 22 ml 20 %ige Natronlauge zu. Nach dem Abkühlen trennt man die wäßrige Schicht ab, versetzt die organische Phase mit weiteren.

300 g Wasser und rührt nochmals 4 Stunden bei 160 °C. Die abgetrennte organische Phase wird destilliert. Man erhält 121 g Destillat. Es verbleibt ein Rückstand von 7,3 g.

Das Destillat ist wie folgt zusammengesetzt :

| | |
|---|---|
| Unbekannte Anteile | 0,7 % |
| 3-Phenoxy-toluol | 21,0 % |
| kernchlorierte Anteile | 2,8 % |
| 3-Phenoxy-benzaldehyd | 13,3 % |
| 3-Phenoxy-benzylchlorid | 0,3 % |
| Unbekannte Anteile | 1,6 % |
| 3-Phenoxy-benzylalkohole | 59,8 % |
| Unbekannte Anteile | 0,5 % |

Bezogen auf eingesetztes 3-Phenoxy-benzylchlorid beträgt die Ausbeute an 3-Phenoxy-benzylalkohol 86 % des theoretischen Umsatzes.

**Ansprüche**

1. Verfahren zur Herstellung von 3-Phenoxy-benzaldehyden aus 3-Phenoxy-benzylalkoholen, dadurch gekennzeichnet, daß man 3-Phenoxy-benzylalkohole der Formel

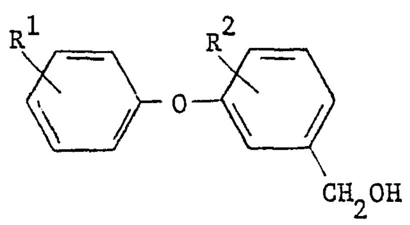

worin $R^1$ und $R^2$ gleich oder verschieden sind und für Wasserstoff oder Halogen stehen, in Gegenwart von wässriger Schwefelsäure im Temperaturbereich von 50 bis 125 °C mit einer wässrigen Lösung eines Dichromats oxydiert.

2. Verfahren nach Anspruch 1, dadurch gekennzeichnet, daß man die Umsetzung im Temperaturbereich von 80 bis 100 °C durchführt.

3. Verfahren nach den Ansprüchen 1 und 2, dadurch gekennzeichnet, daß man die Oxydation in Gegenwart von 2,5 bis 10 Mol Schwefelsäure, bezogen auf 1 Mol des Dichromats durchführt.

4. Verfahren nach den Ansprüchen 1 bis 3, dadurch gekennzeichnet, daß man 10 bis 70 Gew.-%ige Schwefelsäure verwendet.

5. Verfahren nach den Ansprüchen 1 bis 4, dadurch gekennzeichnet, daß man als Dichromat ein Alkalidichromat verwendet.

6. Verfahren nach den Ansprüchen 1 bis 5, dadurch gekennzeichnet, daß man 0,25 bis 0,4 Mol des Dichromats, bezogen auf 1 Mol des Benzylalkohols einsetzt.

7. Verfahren nach den Ansprüchen 1 bis 6, dadurch gekennzeichnet, daß man eine 10 Gew.-% bis gesättigte wäßrige Lösung des Dichromats einsetzt.

8. Verfahren nach den Ansprüchen 1 bis 7, dadurch gekennzeichnet, daß es in einem inerten, nicht wasserlöslichen Lösungsmittel durchgeführt wird.

9. Verfahren zur Herstellung von 3-Phenoxy-benzaldehyden, dadurch gekennzeichnet, daß man in einer ersten Reaktionsstufe ein Gemisch aus seitenkettenchlorierten 3-Phenoxy-toluolen der Formel

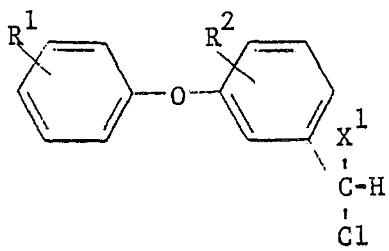

worin $X^1$ für Wasserstoff oder Chlor steht, und $R^1$ und $R^2$ gleich oder verschieden sind und die oben angegebene Bedeutung haben, im Temperaturbereich von 140 bis 210 °C unter Druck hydrolysiert und dann in einer zweiten Reaktionsstufe das Reaktionsgemisch in Gegenwart von wäßriger Schwefelsäure im Temperaturbereich von 50 bis 125 °C mit einer wäßrigen Lösung des Dichromats oxydiert.

**Claims**

1. Process for the preparation of 3-phenoxy-benzaldehydes from 3-phenoxy-benzyl alcohols, characterised in that 3-phenoxy-benzyl alcohols of the formula

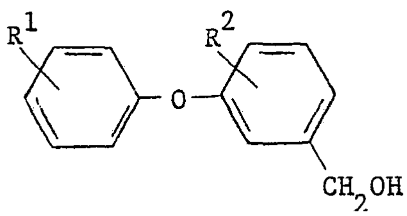

wherein $R^1$ and $R^2$ are identical or different and represent hydrogen or halogen, are oxidised in the presence of aqueous sulphuric acid in the temperature range of 50 to 125 °C with an aqueous solution of a dichromate.

2. Process according to Claim 1, characterised in that the reaction is carried out in the temperature range of 80 to 100 °C.

3. Process according to Claims 1 and 2, characterised in that the oxidation is carried out in the presence of 2.5 to 10 mols of sulphuric acid, based on 1 mol of the dichromate.

4. Process according to Claims 1 to 3, characterised in that 10 to 70 % strength by weight sulphuric acid is used.

5. Process according to Claims 1 to 4, characterised in that the dichromate used is an alkali metal dichromate.

6. Process according to Claims 1 to 5, characterised in that 0.25 to 0.4 mol of the dichromate is employed, based on 1 mol of the benzyl alcohol.

7. Process according to Claims 1 to 6, characterised in that a 10 % by weight to saturated aqueous solution of the dichromate is employed.

8. Process according to Claims 1 to 7, characterised in that it is carried out in an inert solvent which is not soluble in water.

9. Process for the preparation of 3-phenoxy-benzaldehydes, characterised in that a mixture of 3-phenoxy-toluenes, chlorinated in the side chain, of the formula

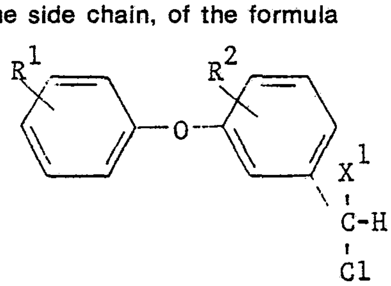

wherein $X^1$ represents hydrogen or chlorine and $R^1$ and $R^2$ are identical or different and have the abovementioned meaning, is hydrolysed in a first reaction stage in the temperature range of 140 to 210 °C under pressure and the reaction mixture is then oxidised, in a second reaction stage, in the presence of aqueous sulphuric acid in the temperature range of 50 to 125 °C with an aqueous solution of the dichromate.

**Revendications**

1. Procédé de préparation de 3-phénoxy-benzaldéhydes à partir d'alcools 3-phénoxy-benzyliques, caractérisé en ce que l'on oxyde des alcools 3-phénoxy-benzyliques de formule

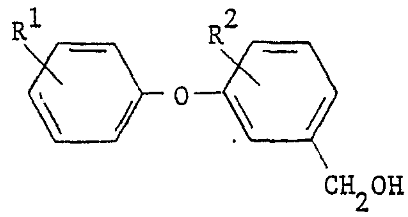

dans laquelle $R^1$ et $R^2$, identiques ou différents, représentent l'hydrogène ou un halogène, en présence d'acide sulfurique aqueux, dans un intervalle de température de 50 à 125 °C, par une solution aqueuse d'un bichromate.

2. Procédé selon la revendication 1, caractérisé en ce que l'on effectue la réaction dans un intervalle de température de 80 à 100 °C.

3. Procédé selon les revendications 1 et 2, caractérisé en ce que l'on effectue l'oxydation en présence de 2,5 à 10 moles d'acide sulfurique pour 1 mole de bichromate.

4. Procédé selon les revendications 1 à 3, caractérisé en ce que l'on utilise de l'acide sulfurique à une concentration de 10 à 70 % en poids.

5. Procédé selon les revendications 1 à 4, caractérisé en ce que l'on utilise en tant que bichromate un bichromate alcalin.

6. Procédé selon les revendications 1 à 5, caractérisé en ce que l'on utilise de 0,25 à 0,4 mole du bichromate pour 1 mole de l'alcool benzylique.

7. Procédé selon les revendications 1 à 6, caractérisé en ce que l'on utilise une solution aqueuse du bichromate dont la concentration va de 10 % en poids jusqu'à la saturation.

8. Procédé selon les revendications 1 à 7, caractérisé en ce qu'il est mis en œuvre dans un solvant inerte non soluble dans l'eau.

9. Procédé de préparation de 3-phénoxy-benzaldéhydes, caractérisé en ce que, dans un premier stade de réaction, on hydrolyse sous pression, dans un intervalle de température de 140 à 210 °C, un mélange de 3-phénoxy-toluènes chlorés en chaîne latérale, de formule

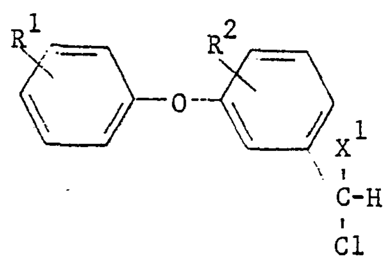

dans laquelle $X^1$ représente l'hydrogène ou le chlore et $R^1$ et $R^2$, identiques ou différents, ont les significations indiquées ci-dessus, puis, dans un deuxième stade de réaction, on oxyde le mélange de réaction en présence d'acide sulfurique aqueux dans l'intervalle de température de 50 à 125 °C par une solution aqueuse du bichromate.